# EUROPEAN PATENT APPLICATION

(11) **EP 4 534 666 A1**
(43) Date of publication of application: **09.04.2025**
(21) Application number: 23811163.7
(22) Date of filing: 26.05.2023
(51) Int. Cl.: C12N 15/10

(54) **METHOD AND REAGENT FOR NUCLEIC ACID EXTRACTION AND PURIFICATION USING POROUS NANOMATERIAL**

(30) Priority: 27.05.2022 CN 202210593931
(71) Applicant: Hangzhou New Horizon Health Technology Co. Ltd., Hangzhou, Zhejiang 310052 (CN)
(72) Inventor: LIU, Jun, Hangzhou, Zhejiang 310052 (CN); DING, Qiutao, Hangzhou, Zhejiang 310052 (CN); CHENG, Yanchao, Hangzhou, Zhejiang 310052 (CN); JIA, Xinye, Hangzhou, Zhejiang 310052 (CN); HUANG, Yuchen, Hangzhou, Zhejiang 310052 (CN); MENG, Xin, Hangzhou, Zhejiang 310052 (CN); ZHANG, Qionglu, Hangzhou, Zhejiang 310052 (CN); CHEN, Chengtong, Hangzhou, Zhejiang 310052 (CN); SHI, Xiaoyu, Hangzhou, Zhejiang 310052 (CN)
(74) Representative: Cooley (UK) LLP
(86) International application number: PCT/CN2023/096451
(87) International publication number: WO 2023/227093

(57) **Abstract**

A method for extracting nucleic acids from a biological sample (such as plasma) using nanopores in a porous material. The method induces nucleic acids into nanopores of the porous material to achieve separation and purification of nucleic acids. The method also has the advantage that the bound nucleic acids will not separate from the pores under separation and cleaning conditions, and thus has good application prospects. The material containing nanopores can have added paramagnetic cores (for example, magnetic microspheres of ferroferric oxide) and be applied to a full-automatic nucleic acid extraction workstation to improve nucleic acid extraction efficiency.

## Description

### Field of the Invention

The present invention relates to a method for extracting nucleic acids from a biological sample using nanopores in a porous material.

### Cross Reference to Related Applications

This application claims priority to the Chinese Patent Application No. 202210593931.1 filed on May 27, 2022, and the content of said patent application is incorporated herein by reference in its entirety.

### Background Art

Liquid autopsy is often considered to be a non-invasive detection method in medical diagnoses and disease screening. Blood test is a routine test in a center of routine physical examination. In many body fluids (including blood), extracellular microRNAs or miRNAs are found to be highly stable and quantitative molecules. miRNAs are non-coding RNAs from cleaving hairpin structure-containing transcripts from endogenous generation by cells with a typical length of 18 to 25 nucleotides. Research has shown that miRNA inhibits protein synthesis mainly by causing 3' UTR of a target mRNA to be completely degraded after transcription or causing it to decrease at the translation level after incomplete complementary binding; however, part of miRNAs can also bind to 5' UTR or the coding region to function. Functions of miRNA do not just occur inside cells that are regulated by endogenously generated miRNA. It's been found in research by a number of research teams that stable miRNA is present in serum and plasma and constitutes a part of nucleic acid in the circulatory system. Circulatory miRNA has been found in body fluids such as serum, plasma, saliva, and urine with the following three main sources: one, passive release from damaged cells or damaged tissues; two, active secretion by exosomes; and three, secretion after binding with RNA-binding proteins instead of being encapsulated by exosomes. At present, existing miRNA separation and purification techniques are mainly Trizol-based phenol-chloroform extraction techniques and column separation techniques. Since miRNA is in stable existence mainly in the form of miRNA-protein complexes, one of the key factors for improving the miRNA extraction efficiency is the separation of miRNA from proteins. The second point regarding improving the miRNA extraction efficiency is that, since the phosphate backbone of nucleic acid is negatively charged, the use of a material with positive charges externally or internally can effectively adsorb and separate miRNA, or the static electromagnetic force in a high-concentration chaotropic salt-shielding solution is used to destroy the hydration layer on the surface of a negative charge-containing material and miRNA, thereby promoting the adsorption between the material and miRNA through hydrogen bond and van der Waals force. Since 2000, mesoporous silicon dioxide nanoparticles have been extensively used in research on pharmaceutical vectors. Due to the characteristics that the special pore structure of mesoporous silicon dioxide nanoparticles has high specific surface area and high pore volume. The branch state of a porous material is of a 3D chain-shaped structure, and a large quantity of unsaturated residue bonds and hydroxy groups in various bonding states exist on the surface thereof. What are currently used include inorganic non-metal porous materials with nanopores (for example, mesoporous silicon dioxide and porous titanium dioxide) and metal organic framework materials (for example, zeolitic imidazolate framework material ZIF-8), which are commonly used in drug administration after being loaded with drugs but haven't played a role in nucleic acid extraction.

### Summary of the Invention

Specifically, the present invention solves technical problems existing in the prior art through the following technical solutions.
Item 1. A kit for nucleic acid extraction, comprising a magnetic porous material, the magnetic porous material comprising mesoporous silicon dioxide having ferroferric oxide cores, the magnetic porous material not comprising amination modification, and nanopores of mesoporous silicon dioxide having an average pore size of 2 to 7 nm or 1 to 3 nm.
Item 2. The kit according to item 1, which is characterized in that the average particle size of the magnetic porous material is 100 to 600 nm.
Item 3. A method for extracting nucleic acid from a sample, which is characterized in that the kit according to item 1 or 2 is used for extracting nucleic acid from the sample, and the nucleic acid comprises miRNA.
Item 4. A method for extracting nucleic acid from a sample, which is characterized in that a porous material containing nanopores is used for nucleic acid extraction, comprising:
   (a) optionally, dispersing and activating the porous material; preferably, the dispersing and activating comprising adding a weak polar solvent A and then dispersing by means of ultrasonic shaking;
   (b) optionally, preparing a preserving liquid of the porous material;
   (c) mixing the porous material and the sample into a mixed solution, and then incubating; optionally, the mixed solution further comprising a lysis and binding solution;
   (d) optionally, adding a weak polar solvent B into the mixed solution, mixing, and then removing the solution portion;
   (e) optionally, adding the lysis and binding solution and a weak polar solvent C into the porous material, mixing, and then removing the solution portion;
   (f) optionally, adding a cleaning solution into the porous material once or multiple times, mixing, and then removing the solution portion; and
   (g) adding an eluting solution into the porous material, incubating, and then separating, thereby extracting the nucleic acid into the eluting solution.
Item 5. The method according to item 4, which is characterized in that the porous material is a solid material with nanopores or a material with multi-level nanopores.
Item 6. The method according to item 4 or 5, which is characterized in that the porous material comprises a porous non-metal monomer material, a porous non-metal oxide material, a porous metal oxide material, a metal-organic framework (MOF) material, or a composite material of a kernel-shell structure with the kernel being a magnetic or non-magnetic inorganic oxide component and the shell having nanopores.
Item 7. The method according to any one of items 4 to 6, which is characterized in that the porous material comprises a non-silicon-based mesoporous material, a non-metal oxide material, a metal oxide material, an MOF material, or a composite material.
Item 8. The method according to any one of items 4 to 7, which is characterized in that the porous material comprises mesoporous nitrogen-doped carbon, mesoporous silicon dioxide, mesoporous titanium dioxide, ZIF-8, and MOF-74.
Item 9. The method according to any one of items 4 to 8, which is characterized in that the porous material comprises mesoporous silicon dioxide.
Item 10. The method according to item 9, which is characterized in that the porous material comprises mesoporous silicon dioxide with ferroferric oxide cores.
Item 11. The method according to any one of items 4 to 10, which is characterized in that the porous material does not have extra amination modification.
Item 12. The method according to any one of items 4 to 11, which is characterized in that the porous material does not have amination modification.
Item 13. The method according to any one of items 4 to 12, which is characterized in that the porous material has hydroxyl modification or no modification.
Item 14. The method according to any one of items 4 to 13, which is characterized in that the porous material has no modification.
Item 15. The method according to any one of items 4 to 14, which is characterized in that the average particle size of the porous material is 10-1,000 nm, 10-800 nm, 10-600 nm, 10-400 nm, 10-200 nm, 200-400 nm, 400-600 nm, 300-700 nm, 200-800 nm, or 200-1000 nm.
Item 16. The method according to any one of items 4 to 15, which is characterized in that the average particle size of the porous material is about 500 nm, 400-600 nm, or 300-700 nm.
Item 16.1. The method according to any one of items 4 to 16, which is characterized in that the average particle size of the porous material is 100-600 nm.
Item 16.2. The method according to any one of items 4 to 15, which is characterized in that the average particle size of the porous material is about 200 nm, 150-250 nm, or 100-300 nm.
Item 16.3. The method according to any one of items 4 to 16, which is characterized in that the average particle size of the porous material is 200-400 nm.
Item 17. The method according to any one of items 4 to 16.3, which is characterized in that the average pore size of nanopores of the porous material is 0.1-50 nm; preferably, the average pore size is 0.1-20 nm; more preferably, the average pore size is 1-10 nm; and more preferably, the average pore size is 2-7 nm or 1-3 nm.
Item 18. The method according to any one of items 4 to 17, which is characterized in that the average pore size of nanopores of the porous material is 2-7 nm.
Item 19. The method according to any one of items 4 to 18, which is characterized in that the average pore size of nanopores of the porous material is 1-3 nm.
Item 20. The method according to any one of items 4 to 19, which is characterized in that the porous material encapsulates magnetic microspheres.
Item 21. The method according to item 20, which is characterized in that the material of the magnetic microspheres comprises one or more of ferroferric oxide, ferric oxide, manganese oxide, and manganomanganic oxide; and preferably, it comprises ferroferric oxide.
Item 22. The method according to any one of items 4 to 21, which is characterized in that the nucleic acid comprises DNA and/or RNA.
Item 23. The method according to any one of items 4 to 22, which is characterized in that the nucleic acid comprises miRNA.
Item 24. The method according to any one of items 4 to 23, which is characterized in that the length of the nucleic acid is shorter than 200 nucleotides; preferably, shorter than 100 nucleotides; more preferably, shorter than 50 nucleotides; and more preferably, shorter than 30 nucleotides.
Item 25. The method according to any one of items 4 to 24, which is characterized in that the length of the nucleic acid is 10-200 nucleotides; preferably, 10-100 nucleotides; more preferably, 10-50 nucleotides; more preferably, 10-30 nucleotides; and more preferably, 15-30 nucleotides.
Item 26. The method according to any one of items 4 to 25, which is characterized in that the sample is serum, plasma, saliva, urine, a biological tissue, a tissue homogenate, or a mixture thereof; and preferably, the sample is serum or plasma.
Item 27. The method according to any one of items 4 to 26, which is characterized in that the method comprises (a) the dispersing and activating step; preferably, the dispersing and activating step comprises adding the weak polar solvent A into the porous material, and then dispersing by means of ultrasonic shaking; preferably, the weak polar solvent A is methanol, ethanol, isopropanol, acetone, or any mixture thereof; and more preferably, the weak polar solvent A is ethanol.
Item 28. The method according to any one of items 4 to 27, which is characterized in that the solution portion is removed after the step (a), a salt solution is added into the porous material, and then the preserving liquid of the porous material is obtained by means of ultrasonic shaking or vortex shaking; preferably, the ingredient content of the salt solution is: guanidine thiocyanate, guanidine hydrochloride, or a mixture of the two with the final concentration at 1-5 mol/L, polyethylene glycol with the molecular weight of 200-8,000 and the final concentration at 0% to 20% (w/v), and the pH of the salt solution is in a range of 3-8; and more preferably, the pH of the salt solution is in a range of 5-7.
Item 29. The method according to any one of items 4 to 28, which is characterized in that the mixed solution of the porous material and the sample further comprises a lysis and binding solution, and the lysis and binding solution comprises guanidine thiocyanate, guanidine hydrochloride, or a mixture of the two with the final concentration at 1-5 mol/L, and preferably, comprises guanidine thiocyanate with the final concentration at 2-4, 2.5-3.5, or about 3 mol/L.
Item 30. The method according to item 29, which is characterized in that the lysis and binding solution comprises guanidine thiocyanate with the final concentration at about 3 mol/L.
Item 31. The method according to item 29 or 30, which is characterized in that the lysis and binding solution comprises sodium chloride with the final concentration at 0.01-1.60 mol/L, 0.1-1.0 mol/L, 0.5-1.0 mol/L, or 0.01-0.60 mol/L, and preferably sodium chloride at 0.2-0.5 mol/L, 0.3-0.7 mol/L, or 0.5-1.0 mol/L.
Item 32. The method according to any one of items 29 to 31, which is characterized in that the lysis and binding solution comprises sodium dodecyl sulfate with the final concentration at 0.1% to 5.0% (w/v), Tween-20 with the final concentration at 1% to 10% (v/v), sodium citrate or tris(hydroxymethyl)aminomethane with the final concentration at 0.01-0.10 mol/L, and one or two selected from ethylenediaminetetraacetic acid and ethylenediaminetetraacetic acid disodium salt with the final concentration at 0.02-0.50 mol/L.
Item 33. The method according to any one of items 4 to 32, which is characterized in that the ratio of the lysis and binding solution to the sample is 0.5:1.0 (v/v)-3.0:1.0 (v/v); more preferably, 1.0:1.0 (v/v)-2.2:1.0 (v/v); and more preferably, 1.0:1.0 (v/v)-1.8:1.0 (v/v).
Item 34. The method according to any one of items 4 to 33, which is characterized in that the mixed solution of the porous material and the sample further comprises proteinase K, and preferably, the final concentration of the proteinase K is 0.1-2.0 mg/mL.
Item 35. The method according to any one of items 4 to 34, which is characterized in that the ratio of the porous material preserving liquid to the sample is 0.1:1.0 (v/v)-2.0:1.0 (v/v); and preferably, 0.20:1.00 (v/v)-0.75:1.00 (v/v).
Item 36. The method according to any one of items 4 to 35, which is characterized in that the porous material comprises mesoporous silicon dioxide, the sample is plasma, and the ratio of mesoporous silicon dioxide to plasma is in a range of 2 mg:1 mL-60 mg:1 mL.
Item 37. The method according to any one of items 4 to 36, which is characterized in that the incubation temperature in the step (c) is 22-80°C, preferably 50-65°C.
Item 38. The method according to any one of items 4 to 37, which is characterized in that the incubation time in the step (c) is 0-120 min, 0-90 min, 0-60 min, 0-50 min, 0-40 min, 0-30 min, or 0-20 min; preferably, 5-120 min; and more preferably, 10-20 min.
Item 39. The method according to any one of items 4 to 38, which is characterized in that the method comprises the step (d); preferably, the final concentration of the weak polar solvent B is 20%-80% (v/v); and preferably, the weak polar solvent B is ethanol, isopropanol, or a mixed solution thereof.
Item 40. The method according to any one of items 4 to 39, wherein the method comprises the step (e); preferably, the weak polar solvent C is ethanol, isopropanol, or a mixed solution thereof; preferably, the ratio of the lysis and binding solution to the weak polar solvent C is 0.3:1.0 (v/v)-4:1 (v/v), and more preferably 1:1 (v/v)-2:1 (v/v); and preferably, the method for removing the solution portion is removing the solution portion after retention using a centrifuge column and/or adsorbing the porous material using a magnetic medium.
Item 41. The method according to any one of items 4 to 40, which is characterized in that the method comprises the step (f); preferably, the cleaning solution is a mixed solution of ethanol and nuclease-free water, wherein the ethanol concentration is 50%-80%, and more preferably 60%-80%; and preferably, the cleaning solution is used to clean for at least two times.
Item 42. The method according to any one of items 4 to 41, which is characterized in that the eluting solution in the step (g) comprises a 10-100 mM tris(hydroxymethyl)aminomethane solution (pH7.0-8.0), a 10-100 mM tris(hydroxymethyl)aminomethane and 10-100 mM ethylenediaminetetraacetic acid solution (pH7.0-8.0), nuclease-free water, a 0.05%-2.00% (v/v) diethyl pyrocarbonate aqueous solution, or any combination thereof; preferably, the incubation time is 1-5 min; and preferably, the separation method is centrifugation and/or using a magnetic medium for adsorbing the porous material.
Item 43. A kit for nucleic acid extraction, comprising a porous material having nanopores.
Item 44. The kit according to item 43, which is characterized in that the porous material is a solid material with nanopores or a material with multi-level nanopores.
Item 45. The kit according to item 43 or 44, which is characterized in that the porous material comprises a porous non-metal monomer material, a porous non-metal oxide material, a porous metal oxide material, a metal-organic framework (MOF) material, or a composite material of a kernel-shell structure with the kernel being a magnetic or non-magnetic inorganic oxide component and the shell having nanopores.
Item 46. The kit according to any one of items 43 to 45, which is characterized in that the porous material comprises a non-silicon-based mesoporous material, a non-metal oxide material, a metal oxide material, an MOF material, or a composite material.
Item 47. The kit according to any one of items 43 to 46, which is characterized in that the porous material comprises mesoporous nitrogen-doped carbon, mesoporous silicon dioxide, mesoporous titanium dioxide, ZIF-8, and MOF-74.
Item 48. The kit according to any one of items 43 to 47, which is characterized in that the porous material comprises mesoporous silicon dioxide.
Item 49. The kit according to item 48, which is characterized in that the porous material comprises mesoporous silicon dioxide with ferroferric oxide cores.
Item 50. The kit according to any one of items 43 to 49, which is characterized in that the porous material does not have extra amination modification.
Item 51. The kit according to any one of items 43 to 50, which is characterized in that the porous material does not have amination modification.
Item 52. The kit according to any one of items 43 to 51, which is characterized in that the porous material has hydroxyl modification or no modification.
Item 53. The kit according to any one of items 43 to 52, which is characterized in that the porous material has no modification.
Item 54. The kit according to any one of items 43 to 53, which is characterized in that the average particle size of the porous material is 10-1,000 nm, 10-800 nm, 10-600 nm, 10-400 nm, 10-200 nm, 200-400 nm, 400-600 nm, 300-700 nm, 200-800 nm, or 200-1000 nm.
Item 55. The kit according to any one of items 43 to 54, which is characterized in that the average particle size of the porous material is about 500 nm, 400-600 nm, or 300-700 nm.
Item 55.1. The kit according to any one of items 43 to 55, which is characterized in that the average particle size of the porous material is 100-600 nm.
Item 55.2. The kit according to any one of items 43 to 54, which is characterized in that the average particle size of the porous material is about 200 nm, 150-250 nm, or 100-300 nm.
Item 55.3. The kit according to any one of items 43 to 55, which is characterized in that the average particle size of the porous material is 200-400 nm.
Item 56. The kit according to any one of items 43 to 55.3, which is characterized in that the average pore size of nanopores of the porous material is 0.1-50 nm; preferably, the average pore size is 0.1-20 nm; more preferably, the average pore size is 1-10 nm; and more preferably, the average pore size is 2-7 nm or 1-3 nm.
Item 57. The kit according to any one of items 43 to 56, which is characterized in that the average pore size of nanopores of the porous material is 2-7 nm.
Item 58. The kit according to any one of items 43 to 57, which is characterized in that the average pore size of nanopores of the porous material is 1-3 nm.
Item 59. The kit according to any one of items 43 to 58, which is characterized in that the porous material encapsulates magnetic microspheres.
Item 60. The kit according to item 59, which is characterized in that the material of the magnetic microspheres comprises one or more of ferroferric oxide, ferric oxide, manganese oxide, and manganomanganic oxide; and preferably, it comprises ferroferric oxide.
Item 61. The kit according to any one of items 43 to 60, which is characterized in that the nucleic acid comprises DNA and/or RNA.
Item 62. The kit according to any one of items 43 to 61, which is characterized in that the nucleic acid comprises miRNA.
Item 63. The kit according to any one of items 43 to 62, which is characterized in that the length of the nucleic acid is shorter than 200 nucleotides; preferably, shorter than 100 nucleotides; more preferably, shorter than 50 nucleotides; and more preferably, shorter than 30 nucleotides.
Item 64. The kit according to any one of items 43 to 63, which is characterized in that the length of the nucleic acid is 10-200 nucleotides; preferably, 10-100 nucleotides; more preferably, 10-50 nucleotides; more preferably, 10-30 nucleotides; and more preferably, 15-30 nucleotides.
Item 65. The kit according to any one of items 43 to 64, which is characterized in that samples targeted by the kit include serum, plasma, saliva, urine, a biological tissue, a tissue homogenate, or a mixture thereof; and preferably, the sample is serum or plasma.
Item 66. The kit according to any one of items 43 to 65, which is characterized in that the kit comprises a weak polar solvent A; preferably, the weak polar solvent A is methanol, ethanol, isopropanol, acetone, or any mixture thereof; and more preferably, the weak polar solvent A is ethanol.
Item 67. The kit according to any one of items 43 to 66, which is characterized in that the kit comprises a salt solution; preferably, the ingredient content of the salt solution is: guanidine thiocyanate, guanidine hydrochloride, or a mixture of the two with the final concentration at 1-5 mol/L, polyethylene glycol with the molecular weight of 200-8,000 and the final concentration at 0% to 20% (w/v), and the pH of the salt solution is in a range of 3-8; and more preferably, the pH of the salt solution is in a range of 5-7.
Item 68. The kit according to any one of items 43 to 67, which is characterized in that the kit comprises a lysis and binding solution, and the lysis and binding solution comprises guanidine thiocyanate, guanidine hydrochloride, or a mixture of the two with the final concentration at 1-5 mol/L, and preferably, comprises guanidine thiocyanate with the final concentration at 2-4, 2.5-3.5, or about 3 mol/L.
Item 69. The kit according to item 68, which is characterized in that the lysis and binding solution comprises guanidine thiocyanate with the final concentration at about 3 mol/L.
Item 70. The kit according to item 68 or 69, which is characterized in that the lysis and binding solution comprises sodium chloride with the final concentration at 0.01-1.60 mol/L, 0.1-1.0 mol/L, 0.5-1.0 mol/L, or 0.01-0.60 mol/L, and preferably sodium chloride at 0.2-0.5 mol/L, 0.3-0.7 mol/L, or 0.5-1.0 mol/L.
Item 71. The kit according to any one of items 68 to 70, which is characterized in that the lysis and binding solution comprises sodium dodecyl sulfate with the final concentration at 0.1% to 5.0% (w/v), Tween-20 with the final concentration at 1% to 10% (v/v), sodium citrate or tris(hydroxymethyl)aminomethane with the final concentration at 0.01-0.10 mol/L, and one or two selected from ethylenediaminetetraacetic acid and ethylenediaminetetraacetic acid disodium salt with the final concentration at 0.02-0.50 mol/L.
Item 72. The kit according to any one of items 43 to 71, which is characterized in that the kit comprises proteinase K.
Item 73. The kit according to any one of items 43 to 72, which is characterized in that the kit comprises the weak polar solvent B; and preferably, the weak polar solvent B is ethanol, isopropanol, or a mixed solution thereof.
Item 74. The method according to any one of items 43 to 73, which is characterized in that the kit comprises the weak polar solvent C; and preferably, the weak polar solvent C is ethanol, isopropanol, or a mixed solution thereof.
Item 75. The kit according to any one of items 43 to 74, which is characterized in that the kit comprises a cleaning solution; and preferably, the cleaning solution is a mixed solution of ethanol and nuclease-free water, wherein the ethanol concentration is 50%-80%, and more preferably 60%-80%.
Item 76. The kit according to any one of items 43 to 75, which is characterized in that the kit comprises an eluting solution; and preferably, the eluting solution comprises a 10-100 mM tris(hydroxymethyl) aminomethane solution (pH7.0-8.0), a 10-100 mM tris(hydroxymethyl)aminomethane and 10-100 mM ethylenediaminetetraacetic acid solution (pH7.0-8.0), nuclease-free water, a 0.05%-2.00% (v/v) diethyl pyrocarbonate aqueous solution, or any combination thereof.

### Brief Description of the Drawings

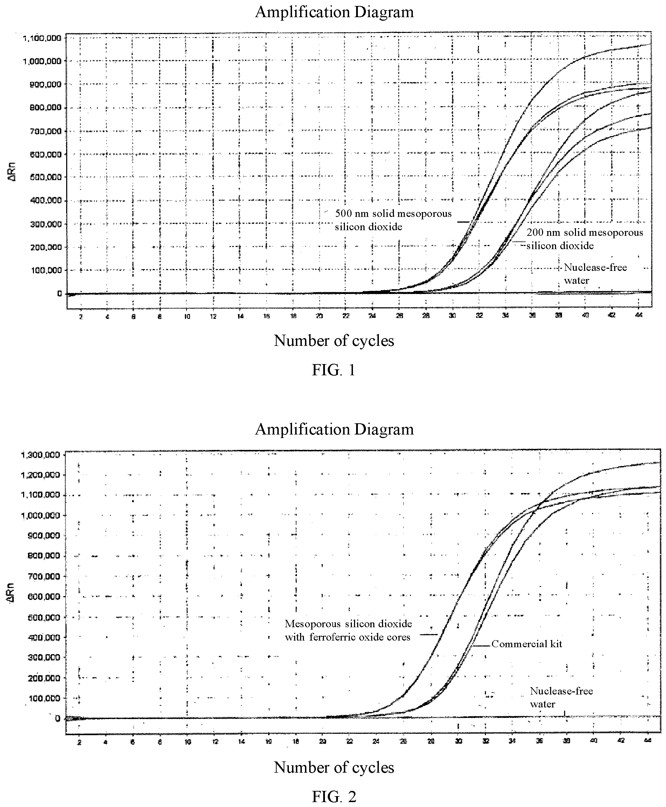

FIG. 1 illustrates DNA amplification curves when using nucleic acid extracted from plasma, by using 10 mg 200 nm solid mesoporous silicon dioxide and 500 nm solid mesoporous silicon dioxide, for RT-qPCR of hsa-miR-122, wherein nuclease-free water is used as the blank control.
FIG. 2 illustrates DNA amplification curves when using nucleic acid extracted from plasma, by using 10 mg mesoporous silicon dioxide with ferroferric oxide cores, for RT-qPCR of hsa-miR-122, wherein the commercial kit, miRNeasy Serum/Plasma Advanced Kit (Cat. 218204) produced by Qiagen, is used, the extraction process is carried out according to the operation manual of the manufacturer, the eluting volume is 0.1 mL, and nuclease-free water is used as the blank control.

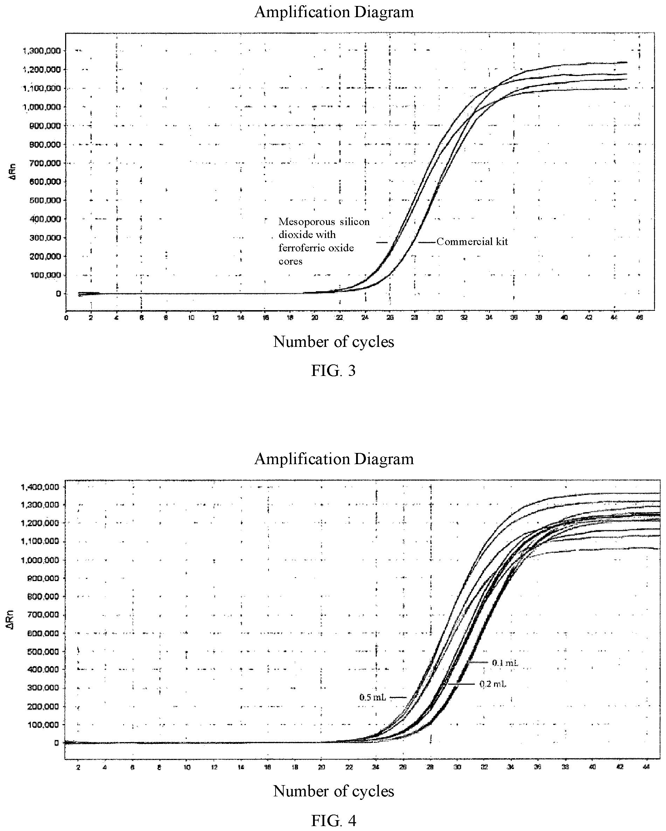

FIG. 3 illustrates DNA amplification curves when using nucleic acid, which is extracted from 0.5 mL suspension containing 1.3×10⁴ cells (human liver cancer cells HepG2) by using 10 mg mesoporous silicon dioxide with ferroferric oxide cores and eluted with 0.1 mL nuclease-free water, for RT-qPCR of hsa-miR-122, wherein the commercial kit, Plasma/Serum Circulating and Exosomal RNA Purification Mini Kit (Cat. 51000) produced by Norgen Biotek, is used, the extraction process is carried out according to the operation manual of the manufacturer, the eluting volume is 0.1 mL, and nuclease-free water is used as the blank control.
FIG. 4 illustrates DNA amplification curves when using nucleic acid, which is extracted from different volumes of plasma by using 10 mg mesoporous silicon dioxide with ferroferric oxide cores, for RT-qPCR of hsa-miR-122, and illustrates amplification curves of nucleic acid from 5 times of repeated extractions of 0.1 mL, 0.2 mL, and 0.5 mL plasma, wherein 0.1 mL plasma and 0.2 mL plasma are added with sterile PBS buffer to 0.5 mL prior to the extraction.

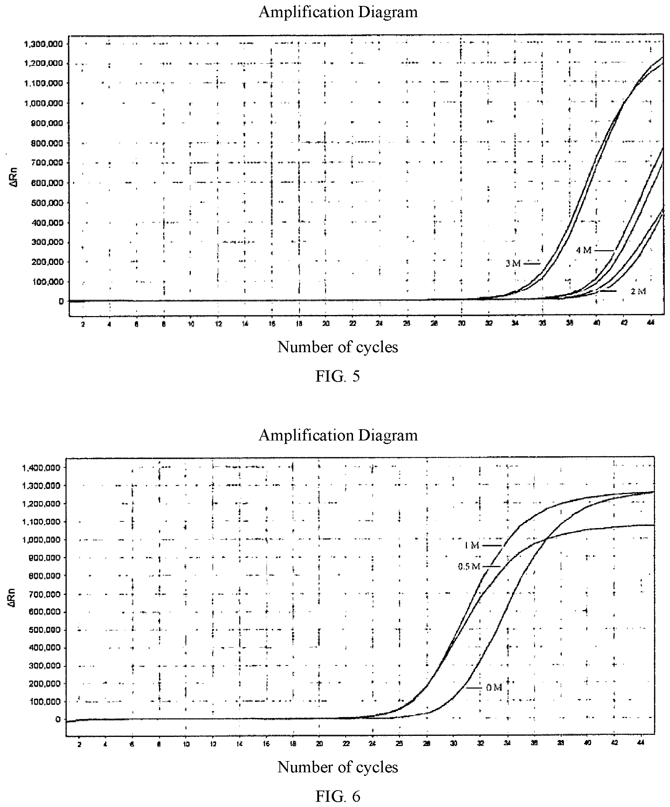

FIG. 5 illustrates the impact of the concentration of guanidine thiocyanate in the lysis and binding solution on the extraction efficiency, and DNA amplification curves when using nucleic acid extracted from plasma, by using 10 mg mesoporous silicon dioxide with ferroferric oxide cores, for RT-qPCR of hsa-miR-122 are shown, wherein the extraction efficiency is the highest when the concentration of guanidine thiocyanate in the lysis and binding solution is 3 M.
FIG. 6 illustrates the impact of the concentration of sodium chloride in the lysis and binding solution on the extraction efficiency, and DNA amplification curves when using nucleic acid extracted from plasma, by using 10 mg mesoporous silicon dioxide with ferroferric oxide cores, for RT-qPCR of hsa-miR-122 are shown, wherein the extraction efficiency is high when the concentration of sodium chloride in the lysis and binding solution is 0.5 M or 1 M.

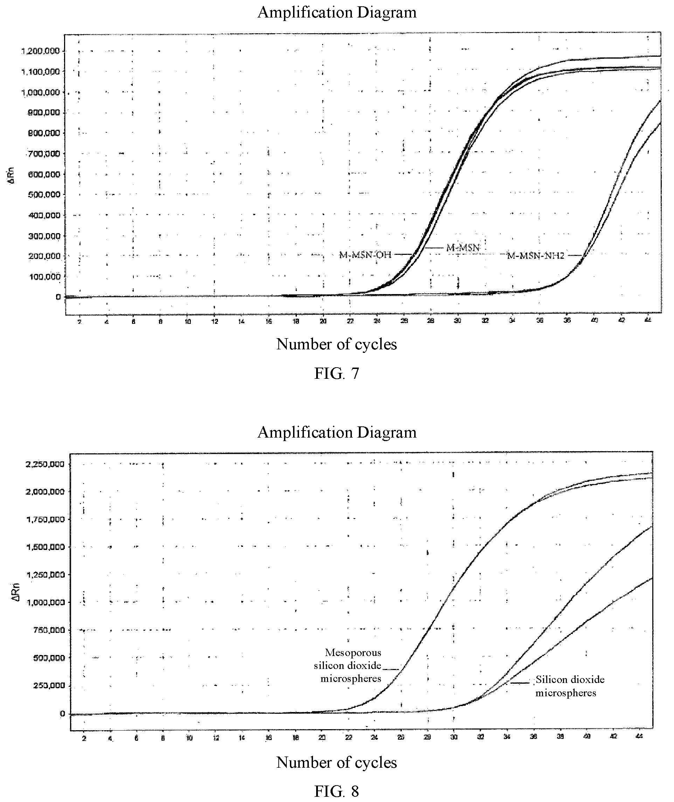

FIG. 7 illustrates the impacts of various modifications to mesoporous silicon dioxide with ferroferric oxide cores on the extraction efficiency, and DNA amplification curves when using nucleic acids extracted from plasma, by using 10 mg mesoporous silicon dioxide with ferroferric oxide cores and with amination modification (M-MSN-NH2) or hydroxyl modification (M-MSN-OH) or no modification (M-MSN), for RT-qPCR of hsa-miR-122 are shown, wherein the results show that the extraction efficiency of mesoporous silicon dioxide with ferroferric oxide cores and with amination modification is far lower than that of mesoporous silicon dioxide with ferroferric oxide cores and with no modification or with hydroxyl modification, and in the meantime, the presence or absence of hydroxyl modification does not lead to a significant difference in the extraction efficiency, indicating that nanopores of the material play the essential role in adsorption of nucleic acid.
FIG. 8 illustrates the difference in the miRNA extraction efficiency between silicon dioxide materials with and without nanopores, wherein the results show that the miRNA extraction efficiency of silicon dioxide with nanopores (mesoporous silicon dioxide microspheres) is significantly better than that of the silicon dioxide material without nanopores (silicon dioxide microspheres), indicating that the effective structure of a material when extracting miRNA is nanopores in the material.

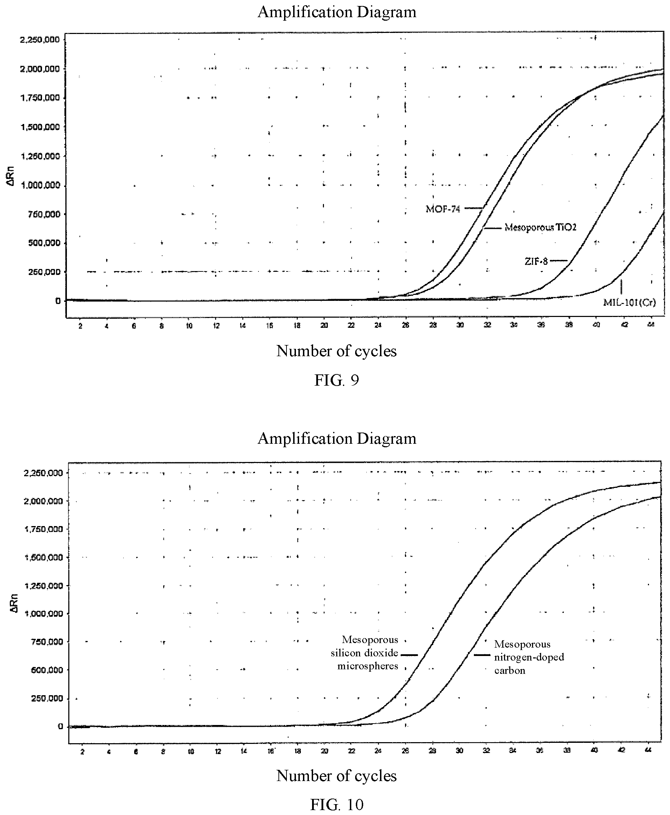

FIG. 9 illustrates the efficiency of nucleic acid extraction when a porous material with nanopores that is not mesoporous silicon dioxide, wherein MOF-74 is comprised of Mg²⁺, 2,5-dihydroxyterephthalic acid (DHTP) and has an average particle size <5 µM and a pore size of 1.2 nm; mesoporous TiO₂ is mesoporous titanium dioxide with a particle size of 10-30 nm; ZIF-8 is comprised of Zn²⁺, 2-methylimidazole acid (C₄H₅N₂-), made using the hydrothermal method, and has an average particle size of 1 µM and a pore size of 0.34-1.16 nm; and MIL-101(Cr) is comprised of Cr²⁺, terephthalic acid (C₈H₅O₄-) and has an average particle size of 100-400 nm and an average pore size of 2.1 nm.
FIG. 10 illustrates the efficiency of extracting miRNA from biological samples using mesoporous silicon dioxide microspheres and mesoporous nitrogen-doped carbon, wherein mesoporous nitrogen-doped carbon has an average particle size of 1 µM and an average pore size of 4.83 nm.

### Detailed Description of the Invention

The present invention provides a method for utilizing nanopores in a porous material to separate and purify nucleic acid molecules from a biological sample. In some embodiments, this method can apply magnetic microspheres encapsulated by a material with nanopores to a fully automated nucleic acid extraction workstation to improve the nucleic acid extraction efficiency, which leads to very high values in commercial applications.

In one aspect of the present invention, a method for extracting nucleic acid from a sample is provided, wherein a porous material containing nanopores is used for nucleic acid extraction, comprising:
(a) optionally, dispersing and activating the porous material; preferably, the dispersing and activating comprising adding a weak polar solvent A and then dispersing by means of ultrasonic shaking;
(b) optionally, preparing a preserving liquid of the porous material;
(c) mixing the porous material and the sample into a mixed solution, and then incubating; optionally, the mixed solution further comprising a lysis and binding solution;
(d) optionally, adding a weak polar solvent B into the mixed solution, mixing, and then removing the solution portion;
(e) optionally, adding the lysis and binding solution and a weak polar solvent C into the porous material, mixing, and then removing the solution portion;
(f) optionally, adding a cleaning solution into the porous material once or multiple times, mixing, and then removing the solution portion; and
(g) adding an eluting solution into the porous material, incubating, and then separating, thereby extracting the nucleic acid into the eluting solution.

In another aspect of the present invention, a kit for nucleic acid extraction is provided, comprising a porous material having nanopores.

In another aspect of the present invention, a use of a porous material having nanopores in nucleic acid extraction is provided.

In some embodiments, the porous material is a solid material with nanopores or a material with multi-level nanopores.

In some embodiments, the porous material comprises a porous non-metal monomer material, a porous non-metal oxide material, a porous metal oxide material, and a metal-organic framework (MOF) material. In some embodiments, the porous material comprises a composite material of a kernel-shell structure with the kernel being an inorganic oxide component and the shell having nanopores. In some embodiments, the kernel is a magnetic inorganic oxide component. In some embodiments, the kernel is a non-magnetic inorganic oxide component.

In some embodiments, the porous material is a non-silicon-based mesoporous material. In some embodiments, the non-silicon-based mesoporous material is mesoporous nitrogen-doped carbon.

In some embodiments, the porous material is a non-metal oxide material. In some embodiments, the non-metal oxide material is mesoporous silicon dioxide.

In some embodiments, the porous material is a metal oxide material. In some embodiments, the metal oxide material is mesoporous titanium dioxide. In some embodiments, the mesoporous titanium dioxide has a particle size of 1-100 nm, 2-80 nm, 3-70 nm, 4-60 nm, 5-60 nm, 6-50 nm, 7-50 nm, 8-40 nm, 9-35 nm, or 10-30 nm. In some embodiments, the mesoporous titanium dioxide has a particle size of 10-30 nm.

In some embodiments, the porous material is an MOF material. In some embodiments, the MOF material is zeolitic imidazolate framework material (ZIF-8) and/or MOF-74.

In some embodiments, the MOF material is zeolitic imidazolate framework material (ZIF-8). In some embodiments, ZIF-8 comprises Zn²⁺ and 2-methylimidazole acid (C₄H₅N₂-). In some embodiments, ZIF-8 is made using the hydrothermal method. In some embodiments, ZIF-8 has an average particle size of 0.1-10 µM, 0.2-5 µM, 0.3-3 µM, 0.5-2 µM, 0.8-1.5 µM, or 0.8-1.2 µM. In some embodiments, ZIF-8 has an average particle size of 0.8-1.2 µM. In some embodiments, ZIF-8 has an average pore size of 0.1-10 nM, 0.2-5 nM, 0.2-4 nM, 0.3-2 nM, or 0.3-1.5 nM. In some embodiments, ZIF-8 has an average pore size of 0.3-1.5 nM.

In some embodiments, the MOF material is MOF-74. In some embodiments, MOF-74 is comprised of Mg²⁺, 2,5-dihydroxyterephthalic acid. In some embodiments, MOF-74 has an average particle size <5 µM. In some embodiments, MOF-74 has an average particle size of 0.01-10 µM, 0.1-5 µM, 0.1-4 µM, 0.1-3 µM, 0.1-2 µM, 0.1-1 µM, 0.2-5 µM, 0.4-5 µM, 0.7-5 µM, or 1-5 µM. In some embodiments, MOF-74 has a pore size of 0.1-10 nm, 0.2-5 nm, 0.3-4 nm, 0.4-3 nm, 0.6-2 nm, 0.8-1.5 nm, or 1.0-1.4 nm. In some embodiments, MOF-74 has a pore size of about 1.2 nm.

In some embodiments, the MOF material is MIL-101(Cr). In some embodiments, MIL-101(Cr) comprises Cr²⁺ and terephthalic acid. In some embodiments, MIL-101(Cr) has an average particle size of 10-2000 nm, 20-1500 nm, 30-1000 nm, 50-800 nm, 60-700 nm, 70-600 nm, 80-500 nm, 90-450 nm, or 100-400 nm. In some embodiments, MIL-101(Cr) has an average pore size of 0.1-50 nm, 0.5-10 nm, 0.7-6 nm, 1-4 nm, 1.5-3 nm, or 1.8-2.5 nm. In some embodiments, MIL-101(Cr) has an average pore size of about 2.1 nm. In some embodiments, the porous material is a material of a kernel-shell structure with the kernel being a magnetic or non-magnetic inorganic oxide component and the shell having nanopores. In some embodiments, the porous material is a composite material containing ferroferric oxide cores.

In some embodiments, the kernel of the porous material is a magnetic microsphere. In some embodiments, the material of the magnetic microsphere comprises one or more of ferroferric oxide, ferric oxide, manganese oxide, and manganomanganic oxide. In some embodiments, the material of the magnetic microsphere comprises ferroferric oxide.

In some embodiments, the composite porous material comprises a mesoporous silicon dioxide shell.

In some embodiments, the porous material does not have extra amination modification. In some embodiments, the porous material does not have amination modification.

In some embodiments, the porous material has hydroxyl modification. In some embodiments, the porous material does not have hydroxyl modification.

In some embodiments, the porous material does not have hydroxyl modification or amination modification. In some embodiments, the porous material has no modification.

In some embodiments, the average particle size of the porous material is 10-1,000 nm, 10-800 nm, 10-600 nm, 10-400 nm, 10-200 nm, 200-400 nm, 400-600 nm, 200-800 nm, or 200-1000 nm. In some embodiments, the average particle size of the porous material is 10-20 nm, 20-30 nm, 30-40 nm, 40-50 nm, 50-70 nm, 70-100 nm, 100-200 nm, 200-300 nm, 300-400 nm, 400-500 nm, 500-700 nm, 700-1000 nm, 10-30 nm, 20-40 nm, 30-50 nm, 40-70 nm, 50-100 nm, 70-200 nm, 100-300 nm, 200-400 nm, 300-500 nm, 400-700 nm, 500-1000 nm, 10-40 nm, 20-50 nm, 30-70 nm, 40-100 nm, 50-200 nm, 70-300 nm, 100-400 nm, 200-500 nm, 300-700 nm, 400-1000 nm, 10-50 nm, 20-70 nm, 30-100 nm, 40-200 nm, 50-300 nm, 70-400 nm, 100-500 nm, 200-700 nm, 300-1000 nm, 10-70 nm, 20-100 nm, 30-200 nm, 40-300 nm, 50-400 nm, 70-500 nm, 100-700 nm, 200-1000 nm, 10-100 nm, 20-200 nm, 30-300 nm, 40-400 nm, 50-500 nm, 70-700 nm, 100-1000 nm, 10-200 nm, 20-300 nm, 30-400 nm, 40-500 nm, 50-700 nm, 70-1000 nm, 10-300 nm, 20-400 nm, 30-500 nm, 40-700 nm, 50-1000 nm, 10-400 nm, 20-500 nm, 30-700 nm, 40-1000 nm, 10-500 nm, 20-700 nm, 30-1000 nm, 10-700 nm, 20-1000 nm, or 10-1000 nm. In some embodiments, the average particle size of the porous material is about 10 nm, 20 nm, 30 nm, 40 nm, 50 nm, 70 nm, 100 nm, 200 nm, 300 nm, 400 nm, 500 nm, 700 nm, or 1000 nm.

In some embodiments, the average particle size of the porous material is 100-600 nm. In some embodiments, the average particle size of the porous material is 100-300 nm. In some embodiments, the average particle size of the porous material is 200-400 nm. In some embodiments, the average particle size of the porous material is 300-500 nm. In some embodiments, the average particle size of the porous material is 400-600 nm.

In some embodiments, the average particle size of the porous material is 100-300 nm, 150-250 nm, or about 200 nm.

In some embodiments, the average particle size of the porous material is 200-400 nm, 250-350 nm, or about 300 nm.

In some embodiments, the average particle size of the porous material is 300-700 nm, 400-600 nm, 450-550 nm, or about 500 nm.

The average particle size of the porous material may be determined using a particle size analyzer.

In some embodiments, the average pore size of nanopores of the porous material is 0.1-50 nm. In some embodiments, the average pore size of nanopores of the porous material is 0.1-0.2 nm, 0.1-0.5 nm, 0.1-1 nm, 0.1-2 nm, 0.1-3 nm, 0.1-4 nm, 0.1-5 nm, 0.1-7 nm, 0.1-10 nm, 0.1-20 nm, 0.1-30 nm, 0.1-40 nm, 0.1-50 nm, 0.2-0.5 nm, 0.2-1 nm, 0.2-2 nm, 0.2-3 nm, 0.2-4 nm, 0.2-5 nm, 0.2-7 nm, 0.2-10 nm, 0.2-20 nm, 0.2-30 nm, 0.2-40 nm, 0.2-50 nm, 0.5-1 nm, 0.5-2 nm, 0.5-3 nm, 0.5-4 nm, 0.5-5 nm, 0.5-7 nm, 0.5-10 nm, 0.5-20 nm, 0.5-30 nm, 0.5-40 nm, 0.5-50 nm, 1-2 nm, 1-3 nm, 1-4 nm, 1-5 nm, 1-7 nm, 1-10 nm, 1-20 nm, 1-30 nm, 1-40 nm, 1-50 nm, 2-3 nm, 2-4 nm, 2-5 nm, 2-7 nm, 2-10 nm, 2-20 nm, 2-30 nm, 2-40 nm, 2-50 nm, 3-4 nm, 3-5 nm, 3-7 nm, 3-10 nm, 3-20 nm, 3-30 nm, 3-40 nm, 3-50 nm, 4-5 nm, 4-7 nm, 4-10 nm, 4-20 nm, 4-30 nm, 4-40 nm, 4-50 nm, 5-7 nm, 5-10 nm, 5-20 nm, 5-30 nm, 5-40 nm, 5-50 nm, 7-10 nm, 7-20 nm, 7-30 nm, 7-40 nm, 7-50 nm, 10-20 nm, 10-30 nm, 10-40 nm, 10-50 nm, 20-30 nm, 20-40 nm, 20-50 nm, 30-40 nm, 30-50 nm, or 40-50 nm. In some embodiments, the average pore size of nanopores is 0.1-20 nm. In some embodiments, the average pore size of nanopores is 1-10 nm. In some embodiments, the average pore size of nanopores is 2-7 nm. In some embodiments, the average pore size of nanopores is 1-3 nm.

The pore size of the porous material may be determined using the BET specific surface area detection method.

In some embodiments, the extracted nucleic acid comprises DNA. In some embodiments, the extracted nucleic acid comprises RNA. In some embodiments, the extracted nucleic acid comprises single-stranded nucleic acid. In some embodiments, the extracted nucleic acid comprises double-stranded nucleic acid. In some embodiments, the extracted nucleic acid is extracellular nucleic acid. In some embodiments, the length of the extracted nucleic acid is shorter than 200 nucleotides. In some embodiments, the length of the extracted nucleic acid is shorter than 100 nucleotides. In some embodiments, the length of the extracted nucleic acid is shorter than 50 nucleotides. In some embodiments, the length of the extracted nucleic acid is shorter than 30 nucleotides.

In some embodiments, the extracted nucleic acid comprises microRNA. microRNA is a type of small endogenous non-coding RNA molecules with a size of 17-25 nucleotides. These miRNAs may typically target one or more mRNAs and regulate gene expression by inhibiting the translation level or breaking the target mRNAs. miRNAs have greater advantages than mRNAs when serving as tumor markers, since they are typically more stable.

miRNA is produced from pri-miRNA, wherein pri-miRNA is processed by RNase III Drosha into a precursor miRNA having a stem-loop structure, and then under the action of Dicer, the precursor miRNA is further cleaved in cytoplasm to produced mature miRNA. During the maturation process, miRNA would produce iso-miRNAs (a miRNA subtype), which may be classified into three mutation types according to various positions: 3', 5', and middle. There are roughly two mechanisms for the production of iso-miRNAs, one is caused by the cleavage position offset of Drosha or Dicer enzyme in the microRNA maturation process, and the other is produced by modification after transcription. Studies have shown that iso-miRNAs are different from mature microRNA in stability and expression abundance, and will regulate different downstream signal paths.

In some embodiments, the length of the extracted nucleic acid is 10-15 nucleotides, 15-20 nucleotides, 20-25 nucleotides, 25-30 nucleotides, 30-40 nucleotides, 40-50 nucleotides, 50-70 nucleotides, 70-100 nucleotides, 10-20 nucleotides, 15-25 nucleotides, 20-30 nucleotides, 25-40 nucleotides, 30-50 nucleotides, 40-70 nucleotides, 50-100 nucleotides, 10-25 nucleotides, 15-30 nucleotides, 20-40 nucleotides, 25-50 nucleotides, 30-70 nucleotides, 40-100 nucleotides, 10-30 nucleotides, 15-40 nucleotides, 20-50 nucleotides, 25-70 nucleotides, 30-100 nucleotides, 10-40 nucleotides, 15-50 nucleotides, 20-70 nucleotides, 25-100 nucleotides, 10-50 nucleotides, 15-70 nucleotides, 20-100 nucleotides, 10-70 nucleotides, 15-100 nucleotides, or 10-100 nucleotides. In some embodiments, the length of the extracted nucleic acid is about 10 nucleotides, 15 nucleotides, 20 nucleotides, 25 nucleotides, 30 nucleotides, 40 nucleotides, 50 nucleotides, 70 nucleotides, or 100 nucleotides. In some embodiments, the length of the extracted nucleic acid is 10-200 nucleotides. In some embodiments, the length of the extracted nucleic acid is 10-100 nucleotides. In some embodiments, the length of the extracted nucleic acid is 10-50 nucleotides. In some embodiments, the length of the extracted nucleic acid is 10-25 nucleotides. In some embodiments, the length of the extracted nucleic acid is 10-30 nucleotides. In some embodiments, the length of the extracted nucleic acid is 15-30 nucleotides.

In some embodiments, the sample is serum, plasma, saliva, urine, a biological tissue, a tissue homogenate, or a mixture thereof. In some embodiments, the sample is serum or plasma.

In some embodiments, the method comprises (a) the dispersing and activating step. In some embodiments, the dispersing and activating step comprises adding the weak polar solvent A into the porous material, and then dispersing by means of ultrasonic shaking. In some embodiments, the weak polar solvent A is methanol, ethanol, isopropanol, acetone, or any mixture thereof. In some embodiments, the weak polar solvent A is ethanol.

In some embodiments, the method comprises removing the solution portion after the step (a), adding a salt solution into the porous material, and then obtaining the preserving liquid of the porous material by means of ultrasonic shaking or vortex shaking. In some embodiments, the kit comprises a salt solution. In some embodiments, the ingredient content of the salt solution is: guanidine thiocyanate, guanidine hydrochloride, or a mixture of the two with the final concentration at 1-5 mol/L, and polyethylene glycol with the molecular weight of 200-8,000 and the final concentration at 0% to 20% (w/v). In some embodiments, the pH of the salt solution is in a range of 3-8. In some embodiments, the pH of the salt solution is in a range of 5-7.

**In** some embodiments, the mixed solution of the porous material and the sample further comprises a lysis and binding solution. **In** some embodiments, the kit comprises a lysis and binding solution. **In** some embodiments, the lysis and binding solution comprises guanidine thiocyanate, guanidine hydrochloride, or a mixture of the two with the final concentration at 1-5 mol/L. **In** some embodiments, the lysis and binding solution comprises guanidine thiocyanate with the final concentration at 2-4, 2.5-3.5, or about 3 mol/L. **In** some embodiments, the lysis and binding solution comprises guanidine thiocyanate with the final concentration at about 3 mol/L. **In** some embodiments, the lysis and binding solution comprises sodium chloride with the final concentration at 0.01-1.60 mol/L, 0.1-1.0 mol/L, 0.5-1.0 mol/L, or 0.01-0.60 mol/L. **In** some embodiments, the lysis and binding solution comprises sodium chloride at 0.2-0.5 mol/L. **In** some embodiments, the lysis and binding solution comprises sodium chloride at 0.3-0.7 mol/L. **In** some embodiments, the lysis and binding solution comprises sodium chloride at 0.5-1.0 mol/L. **In** some embodiments, the lysis and binding solution comprises sodium dodecyl sulfate with the final concentration at 0.1% to 5.0% (w/v), Tween-20 with the final concentration at 1% to 10% (v/v), sodium citrate or tris(hydroxymethyl)aminomethane with the final concentration at 0.01-0.10 mol/L, and one or two selected from ethylenediaminetetraacetic acid and ethylenediaminetetraacetic acid disodium salt with the final concentration at 0.02-0.50 mol/L.

**In** some embodiments, the ratio of the lysis and binding solution to the sample is 0.5:1.0 (v/v)-3.0:1.0 (v/v). **In** some embodiments, the ratio of the lysis and binding solution to the sample is 1.0:1.0 (v/v)-2.2:1.0 (v/v). **In** some embodiments, the ratio of the lysis and binding solution to the sample is 1.0:1.0 (v/v)-1.8:1.0 (v/v).

In some embodiments, the method further comprises separating miRNA in the sample from its corresponding protein. In some embodiments, the kit further comprises a reagent for separating miRNA in the sample from the miRNA-protein complex. In some embodiments, the method/reagent for separation comprises the use of a proteinase. In some embodiments, the proteinase comprises proteinase K.

In some embodiments, the mixed solution of the porous material and the sample further comprises proteinase K. In some embodiments, the kit comprises proteinase K. In some embodiments, the final concentration of the proteinase K is 0.1-2.0 mg/mL. In some embodiments, the final concentration of the proteinase K is 0.1-1.0 mg/mL.

In some embodiments, the ratio of the porous material preserving liquid to the sample is 0.1:1.0 (v/v)-2.0:1.0 (v/v). In some embodiments, the ratio of the porous material preserving liquid to the sample is 0.20:1.00 (v/v)-0.75:1.00 (v/v).

In some embodiments, the porous material comprises mesoporous silicon dioxide, the sample is plasma, and the ratio of mesoporous silicon dioxide to plasma is in a range of 2 mg:1 mL-60 mg:1 mL.

In some embodiments, the incubation temperature in the method step (c) is 22-80°C. In some embodiments, the incubation temperature in the method step (c) is 50-65°C.

In some embodiments, the incubation time in the step (c) is 0-120 min, 0-90 min, 0-60 min, 0-50 min, 0-40 min, 0-30 min, or 0-20 min. In some embodiments, the incubation time in the step (c) is 5-120 min. In some embodiments, the incubation time in the step (c) is 10-20 min.

In some embodiments, the method comprises the step (d). In some embodiments, the kit comprises a weak polar solvent B. In some embodiments, the final concentration of the weak polar solvent B is 20%-80% (v/v). In some embodiments, the weak polar solvent B is ethanol, isopropanol, or a mixed solution thereof.

In some embodiments, the method comprises the step (e). In some embodiments, the kit comprises a weak polar solvent C. In some embodiments, the weak polar solvent C is ethanol, isopropanol, or a mixed solution thereof. In some embodiments, the ratio of the lysis and binding solution to the weak polar solvent C is 0.3:1.0 (v/v)-4:1 (v/v). In some embodiments, the ratio of the lysis and binding solution to the weak polar solvent C is 1:1 (v/v)-2:1 (v/v). In some embodiments, the method for removing the solution portion comprises removing the solution portion after retention using a centrifuge column and/or adsorbing the porous material using a magnetic medium. In some embodiments, the method for removing the solution portion comprises removing the solution portion after retention using a centrifuge column. In some embodiments, the method for removing the solution portion comprises removing the solution portion after adsorbing the porous material using a magnetic medium.

In some embodiments, the method comprises the step (f). In some embodiments, the kit comprises a cleaning solution. In some embodiments, the cleaning solution is a mixed solution of ethanol and nuclease-free water, wherein the ethanol concentration is 50%-80%. In some embodiments, the ethanol concentration is 60%-80%. In some embodiments, the method uses the cleaning solution to clean for at least two times.

In some embodiments, the method comprises the step (g). In some embodiments, the kit comprises an eluting solution. In some embodiments, the eluting solution comprises a 10-100 mM tris(hydroxymethyl)aminomethane solution (pH7.0-8.0), a 10-100 mM tris(hydroxymethyl) aminomethane and 10-100 mM ethylenediaminetetraacetic acid solution (pH7.0-8.0), nuclease-free water, a 0.05%-2.00% (v/v) diethyl pyrocarbonate aqueous solution, or any combination thereof. In some embodiments, the incubation time of the eluting solution is 1-5 min. In some embodiments, the separation method of the eluting solution is centrifugation and/or using a magnetic medium for adsorbing the porous material. In some embodiments, the separation method of the eluting solution is centrifugation. In some embodiments, the separation method of the eluting solution is using a magnetic medium for adsorbing the porous material.

In some embodiments, the present invention adopts the following technical solutions:
A method for extracting nucleic acid from a biological sample using nanopores in a porous material specifically comprises the following steps:
(a) weighing 100 to 500 mg particles of a porous material having nanopores, placing the same in a centrifuge tube, adding 0.1 to 15.0 mL of a weak polar solvent, and causing the material to be evenly dispersed in the weak polar solvent by means of 50-150 W ultrasonic shaking processing; the particle size of the used nanoparticles having nanopores is not limited, and the preferred particle size is 10-1000 nm; the pore size of nanopores of the porous material is 0.1-50 nm, and the preferred pore size is 0.1-20 nm; the porous material having nanopores is preferably a solid material with nanopores or a material with multi-level nanopores, including but not limited to a porous non-metal oxide material, a porous metal oxide material, a metal-organic framework (MOF) material, or a composite material of a kernel-shell structure with the kernel being a magnetic or non-magnetic inorganic oxide component and the shell having nanopores; the type of the porous material includes, but is not limited to, non-metal monomers, metal and non-metal oxides, MOF materials, and relevant composite materials, such as non-silicon-based mesoporous materials like mesoporous nitrogen-doped carbon, non-metal oxides like mesoporous silicon dioxide, metal oxides like microporous titanium dioxide, metal MOF materials like ZIF-8 and MOF-74, and composite materials like mesoporous silicon dioxide with ferroferric oxide cores; the weak polar solvent includes, but is not limited to, one or more of methanol, ethanol, isopropanol, and acetone, and preferably the solvent is ethanol; the magnetic inorganic oxide component includes, but is not limited to, one or more of ferroferric oxide, ferric oxide, manganese oxide, and manganomanganic oxide, and preferably ferroferric oxide;
(b) centrifuging the mixed solution obtained in the step (a) (at 2,000-20,000 RCF), then discarding the supernatant, adding 0.1-10 mL of a salt solution, dispersing the mixed solution by means of ultrasonic shaking (50-150 W) or vortex shaking for later use, and obtaining the preserving liquid; the ingredient content of the salt solution is: one or two of guanidine thiocyanate and guanidine hydrochloride with the final concentration at 1-5 mol/L; polyethylene glycol with the molecular weight of 200-8,000 and the final concentration at 0% to 20% (w/v); and the pH of the salt solution is in a range of 3-7, preferably 5-7;
(c) adding a lysis and binding solution, a proteinase K solution, and the preserving liquid prepared in the step (b) into the biological sample, mixing homogeneously by means of vortex shaking, and leaving the same undisturbed at a certain temperature for a period of time; the biological sample may be one or a mixture of a biological body fluid, a biological tissue, and a tissue homogenate; the final concentration of the added proteinase K is 0.1-1.0 mg/mL; the ratio of the added lysis and binding solution to the biological sample is 0.5:1.0 (v/v)-3.0:1.0 (v/v), preferably 1.0:1.0 (v/v)-1.8:1.0 (v/v); the ingredient content of the lysis and binding solution is: one or two of guanidine thiocyanate and guanidine hydrochloride with the final concentration at 1-5 mol/L; sodium chloride with the final concentration at 0.1-0.6 mol/L; sodium dodecyl sulfate with the final concentration at 0.1% to 5.0% (w/v); Tween-20 with the final concentration at 1% to 10% (v/v); sodium citrate or tris(hydroxymethyl)aminomethane with the final concentration at 0.01-0.10 mol/L; and one or two selected from ethylenediaminetetraacetic acid and ethylenediaminetetraacetic acid disodium salt with the final concentration at 0.01-0.50 mol/L; the ratio of the added preserving solution to the biological sample is 0.1:1.0 (v/v)-2.0:1.0 (v/v), preferably 0.20:1.00 (v/v)-0.75:1.00 (v/v); the temperature is 22-80°C, preferably 50-65°C; and the undisturbed period of time is 5-120 min, preferably 10-20 min;
(d) adding a weak polar solvent with the final concentration at 20% to 80% into the mixed solution from the step (c), mixing homogeneously by means of vortex shaking, and then separating the porous material; the weak polar solvent includes, but is not limited to, one or two of ethanol and isopropanol; and the separation method may be centrifuging at 2,000-20,000 RCF for 1-5 min and then removing the supernatant, and/or using a magnetic medium for adsorbing the porous material and then removing the supernatant;
(e) adding an appropriate amount of the lysis and binding solution used in the step (c) and a weak polar solvent into the porous material in the step (d), mixing homogeneously by means of vortex shaking or dispersing by means of ultrasonic shaking (50-150 W), then adding the same into a nuclease-free centrifuge column, centrifuging for 1-3 min until all the porous material is retained on the filter of the centrifuge column and/or using a magnetic medium for adsorbing the porous material, and then removing the supernatant; the weak polar solvent includes, but is not limited to, one or two of ethanol and isopropanol; and the ratio of the lysis and binding solution to the weak polar solvent is 0.3:1.0 (v/v)-4:1 (v/v), preferably 1:1 (v/v)-2:1 (v/v);
(f) using a cleaning solution to clean the porous material twice, and removing the cleaning solution by means of centrifugation or magnetically separating the porous material after each cleaning; the cleaning solution being a mixed solution of ethanol and nuclease-free water, wherein the ethanol concentration is 50%-80%, preferably 60%-80%; and
(g) adding an appropriate amount of an eluting solution into the porous material in the step (f), incubating for a period of time, and then separating the miRNA-containing extract from the porous material; wherein the eluting solution is one or a combination of a 10-100 mM tris(hydroxymethyl) aminomethane solution (pH7.0-8.0), a 10-100 mM tris(hydroxymethyl)aminomethane and 10-100 mM ethylenediaminetetraacetic acid solution (pH7.0-8.0), nuclease-free water, or a 0.05%-2.00% (v/v) diethyl pyrocarbonate aqueous solution; the incubation time is 1-10 min; and the separation method may be centrifugation and/or using a magnetic medium for adsorbing the porous material, after which the extract product is obtained;

It should be understood that, within the scope of the present invention, the above technical features and various technical features specifically described below (e.g., in the embodiments) of the present invention can all be mutually combined to form new or preferred technical solutions, which will not be elaborated one by one herein.

The advantages of the present invention include but are not limited to:
- The methods and reagents of the present invention induce nucleic acids into nanopores of a porous material, such that the bound nucleic acids are not easy to be separated from the nanopores under separation and cleaning conditions;
- By adding paramagnetic cores, the porous material may be applied to a full-automatic nucleic acid extraction workstation to improve nucleic acid extraction efficiency;
- Having very high extraction efficiency for nucleic acids (for example, short-chain nucleic acids such as microRNA);
- Short operation cycles;
- High material stability; and
- The reagents are relatively safe to the environment and humans.

### Definitions

Unless otherwise defined, all technical and scientific terms used herein have the same meaning as those typically understood by those of ordinary skills in the art. Although any method and material similar or equivalent to the methods and materials described herein may be used when implementing or testing preparations or unit doses herein, some methods and materials need to be described now. Unless otherwise described, technologies adopted or considered herein are standard methods. The materials, methods, and examples are illustrative only, rather than restrictive.

Unless otherwise defined, all technical and scientific terms used herein have the same meaning as those typically understood by those of ordinary skills in the art. The implementation manners described herein may include one or more numerical value ranges (for example, size, concentration, time, and temperature). Unless otherwise clearly specified in the context, a range of numerical values will be construed as including all values within the range, including subsets (one or more) of numerical values in the range until one tenth of the lower limit unit.

As used herein, the article "a," "one," and "the" equivalently relate to the meaning of singular form or plural form, unless otherwise stipulated in the context.

When the term "comprise" is used in the specification or claims of the present invention, unless required by the context, it should be construed as meaning that it includes the stated steps or elements or entities or stated step combinations or element combinations or entity combinations, but does not exclude any other steps or elements or entities or step sets or element sets or entity sets. In order to achieve the objectives of the present invention, the term "be comprised of" is considered to be a preferred embodiment of the term "comprise." The term "comprise" (and related terms such as "contain" or "have" or "include"; "comprising") is not intended to exclude "consisting of" or "consisting essentially of" in some other embodiments (for example, embodiments of any substance composition, composition, method, or process as described herein).

As used herein, unless otherwise specified, the term "or" may be a conjunction or a disjunctive conjunction. As used herein, unless otherwise specified, any embodiment may be combined with any other embodiment.

As used in the present application, the terms "about" and "approximately" are used as equivalents. Any value with or without about/approximately used in the present application means that it covers any normal fluctuation understood by those of ordinary skills in the art. In some embodiments, the term "approximately" or "about" refers to a 10% range in any direction (greater than or smaller than) of the stated reference value, unless otherwise stated or otherwise obviously observable from the context (except for the situation in which the number will exceed 100% of a possible value).

As used herein, unless otherwise specified, the terms "nucleic acid" and "polynucleotide" may be used interchangeably.

### Specific Embodiments

The present invention will be described in detail below with reference to the accompanying drawings and specific embodiments. The provided embodiments are merely for the purpose of describing the present invention, and do not represent limitations to the scope of protection of the present invention. Some non-essential modifications and adjustments made by others according to the concept of the present invention shall still fall within the scope of protection of the present invention. Unless otherwise specified, technical terms used in the present application have the meanings typically understood by those skilled in the art.

Provided that there is no special description, experimental methods in the embodiments of the present invention shall all be carried out according to the methods described in literature in the art or product manuals corresponding to the instruments and reagents. Reagents, consumables, and instruments used in the embodiments of the present invention are all purchased via regular commercial routes if there is no special description.

### Explanations of terms:

As used herein, "v/v" indicates a volume ratio; "w/v" indicates a mass to volume ratio; RCF represents the centrifugal force g. miRNA means micro nucleotide. △Rn represents relative fluorescence value.

Plasma in the following embodiments is from sample 1 and sample 2. Sample 1 (30 years old) and sample 2 (33 years old) are both healthy adult volunteers with no medical history. The cell line is HepG2, which is diluted with PBS and then extracted.

The plasma preparation method: extract 50 mL venous blood from each of sample 1 and sample 2, centrifuge at 1900 RCF and 4°C for 10 min, and obtain about 40 mL of the mixed supernatant. Further centrifuge the supernatant at 3000 RCF and 4°C for 10 min, and obtain about 36 mL of the mixed supernatant.

### Embodiment 1:

A method for extracting nucleic acid from plasma using solid mesoporous silicon dioxide comprises the following steps:
1. Weighing 150 mg solid mesoporous silicon dioxide nanoparticles (200 nm in particle size/500 nm in particle size, and 2-7 nm in pore size), adding 5 mL anhydrous ethanol, applying 100 W ultrasonics for 3 min until complete dispersion, and then mixing homogeneously by means of vortex shaking.
2. Centrifuging the mixed liquid at 2810 RCF for 5 min, and discarding the supernatant.
3. Adding 5 mL 4 M guanidine thiocyanate solution, and then applying ultrasonics (100 W) for 3 min until complete dispersion.
4. Centrifuging the mixed liquid at 2810 RCF for 5 min, and discarding the supernatant.
5. Adding 4.5 mL 4 M guanidine thiocyanate solution, and then applying ultrasonics (100 W) for 3 min until complete dispersion.
6. Adding 0.5 mL 50% autoclaved PEG8000 (w/v), and mixing homogeneously by means of vortex shaking, wherein the preparation of a preserving liquid is completed at this point.
7. Prior to use, heating the preserving liquid at 60°C for 10 min.
8. In a 5 mL nuclease-free centrifuge tube, adding 1.0 mL of a lysis and binding solution (3 M guanidine thiocyanate, 0.5 M sodium chloride, 1% sodium dodecyl sulfate, 5% Tween-20, 25 mM sodium citrate, and 10 mM ethylenediaminetetraacetic acid) into 0.5 mL plasma, shaking to mix homogeneously, adding 0.1 mL proteinase K (20 mg/mL) and then shaking to mix homogeneously again, and incubating at 60°C for 10 min.
9. Adding 0.1 mL of the preserving liquid into the tube, shaking to mix homogeneously, and then incubating at 60°C for 10 min.
10. Adding 2.3 mL anhydrous ethanol, and shaking to mix homogeneously.
11. Centrifuging at 3000 RCF for 3 min, then discarding the supernatant, adding 0.4 mL of the lysis and binding solution, shaking to mix homogeneously, and incubating at 60°C for 10 min.
12. Adding 0.6 mL anhydrous ethanol, and then shaking to mix homogeneously.
13. Transferring the mixture into a nuclease-free centrifuge column with a lid, centrifuging at 10,000 RCF for 2 min, and then discarding the filtrate.
14. Repeating the step 13 until all the liquid has been filtered.
15. Adding 0.5 mL 80% ethanol into the centrifuge column, centrifuging at 10,000 RCF for 2 min, and then discarding the filtrate.
16. Repeating the step 15.
17. Transferring the centrifuge column into a new collection tube, and centrifuging at 10,000 RCF for 3 min.
18. Transferring the centrifuge column into a new 1.5 mL nuclease-free centrifuge tube, and opening up the lid for drying for 2 min.
19. Adding 30 µL nuclease-free water into the centrifuge column, and leaving it undisturbed for 5 min.
20. Centrifuging at 10,000 RCF at room temperature for 2 min, and then adding the filtrate back into the centrifuge column again.
21. Centrifuging at 13,000 RCF at room temperature for 3 min, and then obtaining the extract product.

### Embodiment 2:

A method for extracting nucleic acid from plasma using nanoparticles with ferroferric oxide cores and mesoporous silicon dioxide shells, comprises the following steps:
1. Weighing 200 mg mesoporous silicon dioxide nanoparticles with ferroferric oxide cores (150-200 nm in particle size, and 2-7 nm in pore size), adding 5 mL anhydrous ethanol, applying 100 W ultrasonics for 3 min until complete dispersion, and then mixing homogeneously by means of vortex shaking.
2. Centrifuging the mixed liquid at 2810 RCF for 5 min, and discarding the supernatant.
3. Adding 5 mL 4 M guanidine thiocyanate solution, and then shaking until complete dispersion.
4. Centrifuging the mixed liquid at 2810 RCF for 5 min, and discarding the supernatant.
5. Adding 1.8 mL 4 M guanidine thiocyanate solution, and then shaking until complete dispersion.
6. Adding 0.2 mL 50% autoclaved PEG8000 (w/v), and mixing homogeneously by means of vortex shaking, wherein the preparation of a preserving liquid is completed at this point.
7. Prior to use, heating the preserving liquid at 60°C for 10 min.
8. In a 5 mL nuclease-free centrifuge tube, adding 1.0 mL of a lysis and binding solution (3 M guanidine thiocyanate, 0.5 M sodium chloride, 1% sodium dodecyl sulfate, 5% Tween-20, 25 mM sodium citrate, and 10 mM ethylenediaminetetraacetic acid) into 0.5 mL plasma, shaking to mix homogeneously, adding 0.1 mL proteinase K (20 mg/mL) and then shaking to mix homogeneously again, and incubating at 60°C for 10 min.
9. Adding 0.1 mL of the preserving liquid into the tube, shaking to mix homogeneously, and then incubating at 60°C for 10 min.
10. Adding 2.3 mL anhydrous ethanol, and shaking to mix homogeneously.
11. Placing on a magnetic rack undisturbed for 2 to 5 min until the liquid becomes clear, and then discarding the supernatant.
12. Adding 0.4 mL of the lysis and binding solution, taking the same from the magnetic rack, shaking to mix homogeneously again, and incubating at 60°C for 10 min.
13. Adding 0.6 mL anhydrous ethanol, shaking to mix homogeneously, and then transferring the liquid into a new 2 mL centrifuge tube.
14. Placing on the 2 mL centrifuge tube on the magnetic rack undisturbed for 2 to 5 min until the liquid becomes clear, and then discarding the supernatant.
15. Adding 0.8 mL 80% ethanol into the centrifuge tube, shaking to mix homogeneously, performing short-spin, then placing on the magnetic rack undisturbed for 2 to 5 min until the liquid becomes clear, and then discarding the supernatant.
16. Repeating the step 15.
17. Opening up the lid for drying for 2 min on the magnetic rack.
18. Adding 30 µL nuclease-free water into the centrifuge tube, shaking to mix homogeneously, and leaving it undisturbed for 5 min.
19. Performing short-spin on the centrifuge tube, and then placing it on the magnetic rack until the liquid becomes clear.
20. Transferring the supernatant into a nuclease-free centrifuge tube, and then obtaining the extract product.

Reverse transcription is conducted on hsa-miR-122 using the extract products obtained from **Embodiment 1** (as shown in FIG. 1) and **Embodiment 2** (as shown in FIG. 2 and FIG. 3), and then real-time fluorescence quantitative PCR (qPCR) is conducted. After various volumes of plasma are fixed to 0.5 mL by using the PBS buffer, plasma extraction is performed using mesoporous silicon dioxide with magnetic cores, and detection results are illustrated in FIG. 4. FIG. 5 shows that the nucleic acid extraction efficiency is optimal when 3 M guanidine thiocyanate is used. FIG. 6 shows that the extraction efficiency is higher when a sodium chloride-containing lysis and binding solution is used. FIG. 7 shows that amination modification lowers the nucleic acid extraction efficiency, and hydroxyl modification does not cause a significant difference in the extraction efficiency in comparison with mesoporous silicon dioxide with magnetic cores and having no modification. FIG. 8 illustrates the extraction efficiency of silicon dioxide materials with and without nanopores, where the results show that nanopores in the porous material are the key structure for extracting microRNA. FIG. 9 and FIG. 10 illustrate that the use of the extraction method in Embodiment 1 on different types of porous materials having nanopores can also effectively extract cell-free nucleic acid in plasma.

The reverse transcription kit used in the embodiments and drawings is miRNA 1st Strand cDNA Synthesis Kit (by stem-loop), the manufacturer is Nanjing Vazyme Biotech Co., and its article No. is MR101-02; the qPCR kit used is Taq Pro HS Universal Probe Master Mix, the manufacturer is Nanjing Vazyme Biotech Co., and its article No. is QN113-01.

The reverse transcription reaction system is as follows:
1. Extract product pretreatment
The extract product digesting DNA reaction system is as follows

| | |
|---|---|
| 5×gDNA Wiper Mix | 2.0 µL |
| Extract product | 8.0 µL |

React at 42°C for 2 min, and then place on ice.
2. The reverse transcription reaction system is as follows

| | |
|---|---|
| Product from the previous digestion step | 10.0 µL |
| Nuclease-free water | 5.8 µL |
| Reverse transcription primer (10 µM) | 0.2 µL |
| 10×RT Mix | 2.0 µL |
| HiScript II Enzyme MIX | 2.0 µL |

React at 25°C for 5 min, then react at 50°C for 15 min, and then denature at 85°C for 5 min. Place on ice after the reaction is completed.
The reverse transcription primer sequence is 5'-CTCAACTGGTGTCGTGGAGTCGGCAATTCAGTTGAGAAACCCCA-3' (SEQ ID NO: 1)

3. The qPCR reaction system is as follows

| | |
|---|---|
| Product from the previous reverse transcription step | 2.0 µL |
| Nuclease-free water | 7.0 µL |
| Probe (10 µM) | 0.2 µL |
| Upstream primer (10 µM) | 0.4 µL |
| Downstream primer (10 µM) | 0.4 µL |
| 2×Taq Pro HS universal probe Master Mix | 10.0 µL |

Reaction conditions: denature at 95°C for 30 s; 95°C for 10 s, 60°C for 30 s, and repeat 45 times.
The probe sequence is 5'FAM-TTCAGTTGAGAAACCCCA-3'MGB (SEQ ID NO: 2)
Upstream primer sequence: GCCGAGTGGAGTGTGACAA (SEQ ID NO: 3)
Downstream primer sequence: CTCAACTGGTGTCGTGGAGT (SEQ ID NO: 4)

The content above is a further detailed description of the present invention in combination with specific embodiments, and the specific implementation of the present invention may not be considered as being only limited to such description. Without departing from the concept of the present invention, a simple replacement of a porous material with nanopores, adding surface modifications, replacing internal kernels of a composite material, and replacing, adding, and reducing extraction reagents and ingredients shall all fall within the scope of protection of the present invention.

After reading the content of the present invention taught above, a person skilled in the art may make various changes or modifications to the present invention, and these equivalent forms also fall within the scope defined by the appended claims of the present application.

Although the claims have been set forth as specific combinations of features in the present application, it shall be understood that the scope of the present disclosure also comprises any novel feature or any novel feature combination or any summary thereof explicitly or implicitly disclosed herein, regardless of whether it relates to an invention that is the same as what is currently claimed by any claim, and regardless of whether it mitigates any one or all problems that are the same as the technical problems of the present invention. The applicant hereby provides a notice that during the examination period of the present application or any further application derived therefrom, new claims may be formulated to be such features and/or feature combinations.

A person skilled in the art shall appreciate that, without departing from the whole scope and spirit of the present invention, parts, methods, steps, structures, movements, and coordination described in the present application may be modified (added and/or removed), and the scope and spirit of the present invention encompass such modifications and any and all equivalents thereof.

## Claims

1. A kit for nucleic acid extraction, comprising a magnetic porous material, the magnetic porous material comprising mesoporous silicon dioxide having ferroferric oxide cores, the magnetic porous material not comprising amination modification, and nanopores of mesoporous silicon dioxide having an average pore size of 2 to 7 nm or 1 to 3 nm.

2. The kit according to claim 1, **characterized in that** the average particle size of the magnetic porous material is 100 to 600 nm.

3. A method for extracting nucleic acid from a sample, **characterized in that** the kit according to claim 1 or 2 is used for extracting nucleic acid from the sample, and the nucleic acid comprises miRNA.

4. A method for extracting nucleic acid from a sample, **characterized in that** a porous material containing nanopores is used for nucleic acid extraction, comprising:
(a) optionally, dispersing and activating the porous material; preferably, the dispersing and activating comprising adding a weak polar solvent A and then dispersing by means of ultrasonic shaking;
(b) optionally, preparing a preserving liquid of the porous material;
(c) mixing the porous material and the sample into a mixed solution, and then incubating; optionally, the mixed solution further comprising a lysis and binding solution;
(d) optionally, adding a weak polar solvent B into the mixed solution, mixing, and then removing the solution portion;
(e) optionally, adding the lysis and binding solution and a weak polar solvent C into the porous material, mixing, and then removing the solution portion;
(f) optionally, adding a cleaning solution into the porous material once or multiple times, mixing, and then removing the solution portion; and
(g) adding an eluting solution into the porous material, incubating, and then separating, thereby extracting the nucleic acid into the eluting solution.

5. The method according to claim 4, **characterized in that** the porous material is a solid material with nanopores or a material with multi-level nanopores.

6. The method according to claim 4 or 5, **characterized in that** the porous material comprises a porous non-metal monomer material, a porous non-metal oxide material, a porous metal oxide material, a metal-organic framework (MOF) material, or a composite material of a kernel-shell structure with the kernel being a magnetic or non-magnetic inorganic oxide component and the shell having nanopores.

7. The method according to any one of claims 4 to 6, **characterized in that** the porous material comprises a non-silicon-based mesoporous material, a non-metal oxide material, a metal oxide material, an MOF material, or a composite material.

8. The method according to any one of claims 4 to 7, **characterized in that** the porous material comprises mesoporous nitrogen-doped carbon, mesoporous silicon dioxide, mesoporous titanium dioxide, ZIF-8, and MOF-74.

9. The method according to any one of claims 4 to 8, **characterized in that** the porous material comprises mesoporous silicon dioxide.

10. The method according to claim 9, **characterized in that** the porous material comprises mesoporous silicon dioxide with ferroferric oxide cores.

11. The method according to any one of claims 4 to 10, **characterized in that** the porous material does not have extra amination modification.

12. The method according to any one of claims 4 to 11, **characterized in that** the porous material does not have amination modification.

13. The method according to any one of claims 4 to 12, **characterized in that** the porous material has hydroxyl modification or no modification.

14. The method according to any one of claims 4 to 13, **characterized in that** the porous material has no modification.

15. The method according to any one of claims 4 to 14, **characterized in that** the average particle size of the porous material is 10-1,000 nm, 10-800 nm, 10-600 nm, 10-400 nm, 10-200 nm, 100-600 nm, 200-400 nm, 400-600 nm, 300-700 nm, 200-800 nm, or 200-1000 nm.

16. The method according to any one of claims 4 to 15, **characterized in that** the average particle size of the porous material is 400-600 nm or 300-700 nm.

17. The method according to any one of claims 4 to 16, **characterized in that** the average pore size of nanopores of the porous material is 0.1-50 nm; preferably, the average pore size is 0.1-20 nm; more preferably, the average pore size is 1-10 nm; and more preferably, the average pore size is 2-7 nm or 1-3 nm.

18. The method according to any one of claims 4 to 17, **characterized in that** the average pore size of nanopores of the porous material is 2-7 nm.

19. The method according to any one of claims 4 to 18, **characterized in that** the average pore size of nanopores of the porous material is 1-3 nm.

20. The method according to any one of claims 4 to 19, **characterized in that** the porous material encapsulates magnetic microspheres.

21. The method according to claim 20, **characterized in that** the material of the magnetic microspheres comprises one or more of ferroferric oxide, ferric oxide, manganese oxide, and manganomanganic oxide; and preferably, it comprises ferroferric oxide.

22. The method according to any one of claims 4 to 21, **characterized in that** the nucleic acid comprises DNA and/or RNA.

23. The method according to any one of claims 4 to 22, **characterized in that** the nucleic acid comprises miRNA.

24. The method according to any one of claims 4 to 23, **characterized in that** the length of the nucleic acid is shorter than 200 nucleotides; preferably, shorter than 100 nucleotides; more preferably, shorter than 50 nucleotides; and more preferably, shorter than 30 nucleotides.

25. The method according to any one of claims 4 to 24, **characterized in that** the length of the nucleic acid is 10-200 nucleotides; preferably, 10-100 nucleotides; more preferably, 10-50 nucleotides; more preferably, 10-30 nucleotides; and more preferably, 15-30 nucleotides.

26. The method according to any one of claims 4 to 25, **characterized in that** the sample is serum, plasma, saliva, urine, a biological tissue, a tissue homogenate, or a mixture thereof; and preferably, the sample is serum or plasma.

27. The method according to any one of claims 4 to 26, **characterized in that** the method comprises (a) the dispersing and activating step; preferably, the dispersing and activating step comprises adding the weak polar solvent A into the porous material, and then dispersing by means of ultrasonic shaking; preferably, the weak polar solvent A is methanol, ethanol, isopropanol, acetone, or any mixture thereof; and more preferably, the weak polar solvent A is ethanol.

28. The method according to any one of claims 4 to 27, **characterized in that** the solution portion is removed after the step (a), a salt solution is added into the porous material, and then the preserving liquid of the porous material is obtained by means of ultrasonic shaking or vortex shaking; preferably, the ingredient content of the salt solution is: guanidine thiocyanate, guanidine hydrochloride, or a mixture of the two with the final concentration at 1-5 mol/L, polyethylene glycol with the molecular weight of 200-8,000 and the final concentration at 0% to 20% (w/v), and the pH of the salt solution is in a range of 3-8; and more preferably, the pH of the salt solution is in a range of 5-7.

29. The method according to any one of claims 4 to 28, **characterized in that** the mixed solution of the porous material and the sample further comprises a lysis and binding solution, and the lysis and binding solution comprises guanidine thiocyanate, guanidine hydrochloride, or a mixture of the two with the final concentration at 1-5 mol/L, and preferably, comprises guanidine thiocyanate with the final concentration at 2-4, 2.5-3.5, or about 3 mol/L.

30. The method according to claim 29, **characterized in that** the lysis and binding solution comprises guanidine thiocyanate with the final concentration at about 3 mol/L.

31. The method according to claim 29 or 30, **characterized in that** the lysis and binding solution comprises sodium chloride with the final concentration at 0.01-1.60 mol/L, 0.1-1.0 mol/L, 0.5-1.0 mol/L, or 0.01-0.60 mol/L, and preferably sodium chloride at 0.2-0.5 mol/L, 0.3-0.7 mol/L, or 0.5-1.0 mol/L.

32. The method according to any one of claims 29 to 31, **characterized in that** the lysis and binding solution comprises sodium dodecyl sulfate with the final concentration at 0.1% to 5.0% (w/v), Tween-20 with the final concentration at 1% to 10% (v/v), sodium citrate or tris(hydroxymethyl)aminomethane with the final concentration at 0.01-0.10 mol/L, and one or two selected from ethylenediaminetetraacetic acid and ethylenediaminetetraacetic acid disodium salt with the final concentration at 0.02-0.50 mol/L.

33. The method according to any one of claims 4 to 32, **characterized in that** the ratio of the lysis and binding solution to the sample is 0.5:1.0 (v/v)-3.0:1.0 (v/v); more preferably, 1.0:1.0 (v/v)-2.2:1.0 (v/v); and more preferably, 1.0:1.0 (v/v)-1. 8: 1.0 (v/v).

34. The method according to any one of claims 4 to 33, **characterized in that** the mixed solution of the porous material and the sample further comprises proteinase K, and preferably, the final concentration of the proteinase K is 0.1-2.0 mg/mL.

35. The method according to any one of claims 4 to 34, **characterized in that** the ratio of the porous material preserving liquid to the sample is 0.1:1.0 (v/v)-2.0:1.0 (v/v); and preferably, 0.20:1.00 (v/v)-0.75:1.00 (v/v).

36. The method according to any one of claims 4 to 35, **characterized in that** the porous material comprises mesoporous silicon dioxide, the sample is plasma, and the ratio of mesoporous silicon dioxide to plasma is in a range of 2 mg:1 mL-60 mg:1 mL.

37. The method according to any one of claims 4 to 36, **characterized in that** the incubation temperature in the step (c) is 22-80°C, preferably 50-65°C.

38. The method according to any one of claims 4 to 37, **characterized in that** the incubation time in the step (c) is 0-120 min, 0-90 min, 0-60 min, 0-50 min, 0-40 min, 0-30 min, or 0-20 min; preferably, 5-120 min; and more preferably, 10-20 min.

39. The method according to any one of claims 4 to 38, **characterized in that** the method comprises the step (d); preferably, the final concentration of the weak polar solvent B is 20%-80% (v/v); and preferably, the weak polar solvent B is ethanol, isopropanol, or a mixed solution thereof.

40. The method according to any one of claims 4 to 39, **characterized in that** the method comprises the step (e); preferably, the weak polar solvent C is ethanol, isopropanol, or a mixed solution thereof; preferably, the ratio of the lysis and binding solution to the weak polar solvent C is 0.3:1.0 (v/v)-4:1 (v/v), and more preferably 1:1 (v/v)-2:1 (v/v); and preferably, the method for removing the solution portion is removing the solution portion after retention using a centrifuge column and/or adsorbing the porous material using a magnetic medium.

41. The method according to any one of claims 4 to 40, **characterized in that** the method comprises the step (f); preferably, the cleaning solution is a mixed solution of ethanol and nuclease-free water, wherein the ethanol concentration is 50%-80%, and more preferably 60%-80%; and preferably, the cleaning solution is used to clean for at least two times.

42. The method according to any one of claims 4 to 41, **characterized in that** the eluting solution in the step (g) comprises a 10-100 mM tris(hydroxymethyl)aminomethane solution (pH7.0-8.0), a 10-100 mM tris(hydroxymethyl)aminomethane and 10-100 mM ethylenediaminetetraacetic acid solution (pH7.0-8.0), nuclease-free water, a 0.05%-2.00% (v/v) diethyl pyrocarbonate aqueous solution, or any combination thereof; preferably, the incubation time is 1-5 min; and preferably, the separation method is centrifugation and/or using a magnetic medium for adsorbing the porous material.

43. A kit for nucleic acid extraction, comprising a porous material having nanopores.

44. The kit according to claim 43, **characterized in that** the porous material is a solid material with nanopores or a material with multi-level nanopores.

45. The kit according to claim 43 or 44, **characterized in that** the porous material comprises a porous non-metal monomer material, a porous non-metal oxide material, a porous metal oxide material, a metal-organic framework (MOF) material, or a composite material of a kernel-shell structure with the kernel being a magnetic or non-magnetic inorganic oxide component and the shell having nanopores.

46. The kit according to any one of claims 43 to 45, **characterized in that** the porous material comprises a non-silicon-based mesoporous material, a non-metal oxide material, a metal oxide material, an MOF material, or a composite material.

47. The kit according to any one of claims 43 to 46, **characterized in that** the porous material comprises mesoporous nitrogen-doped carbon, mesoporous silicon dioxide, mesoporous titanium dioxide, ZIF-8, and MOF-74.

48. The kit according to any one of claims 43 to 47, **characterized in that** the porous material comprises mesoporous silicon dioxide.

49. The kit according to claim 48, **characterized in that** the porous material comprises mesoporous silicon dioxide with ferroferric oxide cores.

50. The kit according to any one of claims 43 to 49, **characterized in that** the porous material does not have extra amination modification.

51. The kit according to any one of claims 43 to 50, **characterized in that** the porous material does not have amination modification.

52. The kit according to any one of claims 43 to 51, **characterized in that** the porous material has hydroxyl modification or no modification.

53. The kit according to any one of claims 43 to 52, **characterized in that** the porous material has no modification.

54. The kit according to any one of claims 43 to 53, **characterized in that** the average particle size of the porous material is 10-1,000 nm, 10-800 nm, 10-600 nm, 10-400 nm, 10-200 nm, 100-600 nm, 200-400 nm, 400-600 nm, 300-700 nm, 200-800 nm, or 200-1000 nm.

55. The kit according to any one of claims 43 to 54, **characterized in that** the average particle size of the porous material is 400-600 nm or 300-700 nm.

56. The kit according to any one of claims 43 to 55, **characterized in that** the average pore size of nanopores of the porous material is 0.1-50 nm; preferably, the average pore size is 0.1-20 nm; more preferably, the average pore size is 1-10 nm; and more preferably, the average pore size is 2-7 nm or 1-3 nm.

57. The kit according to any one of claims 43 to 56, **characterized in that** the average pore size of nanopores of the porous material is 2-7 nm.

58. The kit according to any one of claims 43 to 57, **characterized in that** the average pore size of nanopores of the porous material is 1-3 nm.

59. The kit according to any one of claims 43 to 58, **characterized in that** the porous material encapsulates magnetic microspheres.

60. The kit according to claim 59, **characterized in that** the material of the magnetic microspheres comprises one or more of ferroferric oxide, ferric oxide, manganese oxide, and manganomanganic oxide; and preferably, it comprises ferroferric oxide.

61. The kit according to any one of claims 43 to 60, **characterized in that** the nucleic acid comprises DNA and/or RNA.

62. The kit according to any one of claims 43 to 61, **characterized in that** the nucleic acid comprises miRNA.

63. The kit according to any one of claims 43 to 62, **characterized in that** the length of the nucleic acid is shorter than 200 nucleotides; preferably, shorter than 100 nucleotides; more preferably, shorter than 50 nucleotides; and more preferably, shorter than 30 nucleotides.

64. The kit according to any one of claims 43 to 63, **characterized in that** the length of the nucleic acid is 10-200 nucleotides; preferably, 10-100 nucleotides; more preferably, 10-50 nucleotides; more preferably, 10-30 nucleotides; and more preferably, 15-30 nucleotides.

65. The kit according to any one of claims 43 to 64, **characterized in that** samples targeted by the kit include serum, plasma, saliva, urine, a biological tissue, a tissue homogenate, or a mixture thereof; and preferably, the sample is serum or plasma.

66. The kit according to any one of claims 43 to 65, **characterized in that** the kit comprises a weak polar solvent A; preferably, the weak polar solvent A is methanol, ethanol, isopropanol, acetone, or any mixture thereof; and more preferably, the weak polar solvent A is ethanol.

67. The kit according to any one of claims 43 to 66, **characterized in that** the kit comprises a salt solution; preferably, the ingredient content of the salt solution is: guanidine thiocyanate, guanidine hydrochloride, or a mixture of the two with the final concentration at 1-5 mol/L, polyethylene glycol with the molecular weight of 200-8,000 and the final concentration at 0% to 20% (w/v), and the pH of the salt solution is in a range of 3-8; and more preferably, the pH of the salt solution is in a range of 5-7.

68. The kit according to any one of claims 43 to 67, **characterized in that** the kit comprises a lysis and binding solution, and the lysis and binding solution comprises guanidine thiocyanate, guanidine hydrochloride, or a mixture of the two with the final concentration at 1-5 mol/L, and preferably, comprises guanidine thiocyanate with the final concentration at 2-4, 2.5-3.5, or about 3 mol/L.

69. The kit according to claim 68, **characterized in that** the lysis and binding solution comprises guanidine thiocyanate with the final concentration at about 3 mol/L.

70. The kit according to claim 68 or 69, **characterized in that** the lysis and binding solution comprises sodium chloride with the final concentration at 0.01-1.60 mol/L, 0.1-1.0 mol/L, 0.5-1.0 mol/L, or 0.01-0.60 mol/L, and preferably sodium chloride at 0.2-0.5 mol/L, 0.3-0.7 mol/L, or 0.5-1.0 mol/L.

71. The kit according to any one of claims 68 to 70, **characterized in that** the lysis and binding solution comprises sodium dodecyl sulfate with the final concentration at 0.1% to 5.0% (w/v), Tween-20 with the final concentration at 1% to 10% (v/v), sodium citrate or tris(hydroxymethyl)aminomethane with the final concentration at 0.01-0.10 mol/L, and one or two selected from ethylenediaminetetraacetic acid and ethylenediaminetetraacetic acid disodium salt with the final concentration at 0.02-0.50 mol/L.

72. The kit according to any one of claims 43 to 71, **characterized in that** the kit comprises proteinase K.

73. The kit according to any one of claims 43 to 72, **characterized in that** the kit comprises the weak polar solvent B; and preferably, the weak polar solvent B is ethanol, isopropanol, or a mixed solution thereof.

74. The method according to any one of claims 43 to 73, **characterized in that** the kit comprises the weak polar solvent C; and preferably, the weak polar solvent C is ethanol, isopropanol, or a mixed solution thereof.

75. The kit according to any one of claims 43 to 74, **characterized in that** the kit comprises a cleaning solution; and preferably, the cleaning solution is a mixed solution of ethanol and nuclease-free water, wherein the ethanol concentration is 50%-80%, and more preferably 60%-80%.

76. The kit according to any one of claims 43 to 75, **characterized in that** the kit comprises an eluting solution; and preferably, the eluting solution comprises a 10-100 mM tris(hydroxymethyl) aminomethane solution (pH7.0-8.0), a 10-100 mM tris(hydroxymethyl)aminomethane and 10-100 mM ethylenediaminetetraacetic acid solution (pH7.0-8.0), nuclease-free water, a 0.05%-2.00% (v/v) diethyl pyrocarbonate aqueous solution, or any combination thereof.
